Europäisches Patentamt

European Patent Office

Office européen des brevets

(19)

(11) Numéro de publication : **0 426 521 B1**

(12) **FASCICULE DE BREVET EUROPEEN**

(45) Date de publication du fascicule du brevet :
30.03.94 Bulletin 94/13

(51) Int. Cl.$^5$ : **A61K 37/02**

(21) Numéro de dépôt : **90402900.6**

(22) Date de dépôt : **16.10.90**

(54) **L'utilisation de l'interleukine 2 pour le traitement des leucémies.**

(30) Priorité : **17.10.89 FR 8913546**

(43) Date de publication de la demande :
**08.05.91 Bulletin 91/19**

(45) Mention de la délivrance du brevet :
**30.03.94 Bulletin 94/13**

(84) Etats contractants désignés :
**AT BE CH DE DK FR GB IT LI LU NL SE**

(56) Documents cités :
**WO-A-88/06891**
**Proceedings of ASCO, vol. 8 mars 1989, page 198, résumé no. 769 M.K. BRENNER et al.: "Recombinant interleukin infusion in patients with minimal residual hematologic malignancy following cytotoxic chemotherapy (CT) or bone marrow transplantation"**
**BR.J. CANCER, vol. 60, octobre 1989, pages 610-615, The Macmillan Press Ltd. D. J. GOULIEB et al.: "A phase I clinical trial of recombinant interleukin 2 following high dose chemoradiotherapy for haematological malignancy: applicability to the elimation of minimal residual disease".**

(56) Documents cités :
**BR. J. HEAMATOL., vol., 73, no. 2, octobre 1989, pages 168-172 J.A.CARRON et al.: "IL-2 and myelopoiesis: IL 2 induces blast cell proliferation in some cases of acute myeloid leukeamia"**
**FED. PROC., vol. 46,no.4 1987, p.1509, résumé no. 6957; A. ADLER et al.: "Mediated in vitro stimulation of killer cells derived from peripheral blood (PS)/bone marrow (BM) of acuta leukemiapatients"**
**CANCER TREATMENT REVIEW,vol. 16, suppl. A, juin 1989, pages 5-13, Academic Press Ltd; R.K.OLDHAM et al.: "IL-2: a review of current knowledge"**

(73) Titulaire : **ROUSSEL-UCLAF**
**35, Boulevard des Invalides**
**F-75007 Paris (FR)**

(72) Inventeur : **Brandely, Maud**
**135, Boulevard Malesherbes**
**F-75017 Paris (FR)**
Inventeur : **Maraninchi, Dominique**
**115, Le Corbuisier,280 bd Michelet**
**F-13008 Marseille (FR)**

(74) Mandataire : **Bourgouin, André et al**
**Département des Brevets ROUSSEL UCLAF**
**111, route de Noisy B.P. no 9**
**F-93230 Romainville (FR)**

EP 0 426 521 B1

## Description

L'interleukine 2 ($IL_2$) qui est une lymphokine produite par les lymphocytes T activés possède une activité immunomodulatrice et une activité antitumorale décrites par exemple par Fletcher, M. et al. (Lymphokine Research 6 (1987) 47-57), activités qui comprennent en particulier la capacité d'initier la prolifération des lymphocytes T et l'induction de la cytotoxicité des cellules NK (natural killer) et des cellules LAK (lymphokine activated killer). On a observé que l'administration de l'$IL_2$, soit seule à haute dose, soit associée avec des cellules LAK, est capable d'induire la régression de certains cancers établis chez la souris et chez des patients ayant des cancers métastatiques tel que le mélanome, le cancer du rein, le cancer colorectal ou le lymphome non Hodgkinien (Rosenberg, S. A. et al. N. Engl. J. Med. (1987) 316 889-897).

M.K. Brenner et al. (Proceeding of Asco, vol. 8, Mars 1989, page 198, résumé n° 769) a décrit l'administration d'$IL_2$ chez des patients ayant une maladie hématologique résiduelle à la suite d'une chimiothérapie cytotoxique ou d'une transplantation de moelle osseuse et a montré que l'$IL_2$ peut être administrée sans effet indésirable chez ces patients. D.J. Gottlieb et al. (Br. J. Cancer, vol. 60, Octobre 1989, pages 610-615) a décrit l'administration de l'$IL_2$ à la suite de hautes doses de chimio-radiothérapie dans des maladies hématologiques telles que la leucémie myéloïde aigue ou le myélome multiple et a montré le potentiel de l'application de l'$IL_2$ à l'élimination de la maladie résiduelle.

Aucun résultat clinique n'a montré l'efficacité de la thérapie avec l'$IL_2$ seule ou associée avec une immunothérapie adoptive dans les diverses leucémies chroniques ou aiguës connues, notamment dans la leucémie myéloïde aiguë (LMA). Les phénomènes de dysrégulation aboutissant à la prolifération des cellules leucémiques sont complexes. On admet généralement que leur contrôle nécessiterait l'utilisation en association de différents facteurs de différentiation, notamment l'utilisation de l'$IL_2$ avec par exemple un interféron, le facteur de nécrose tumorale, une autre interleukine ou d'autres facteurs de différentiation, comme décrit par exemple pour la leucémie lymphoïde chronique dans la demande de brevet WO 88/06991.

Il a été suggéré puis confirmé que l'$IL_2$ stimule in vitro de façon significative la prolifération de cellules blastiques de malades atteints de LMA (J. A. Carron et J. C. Cawley Br. J. Haematol. 71 (1) (1989) 168).

Or la demanderesse vient d'obtenir de façon inattendue des résultats montrant que l'$IL_2$ seule présente une activité thérapeutique sur la LMA.

L'invention concerne donc l'utilisation d'un polypeptide ayant l'activité de l'interleukine 2 humaine pour préparer une composition pharmaceutique destinée au traitement de la rechute des leucémies ou au traitement préventif de celle-ci. On entend par polypeptide ayant l'activité de l'$IL_2$ humaine, l'$IL_2$ humaine naturelle, l'$IL_2$ humaine recombinante, c'est-à-dire obtenue par la technologie de l'ADN recombinant, par exemple comme cela est décrit par Taniguchi, T. et al. (Nature (1983) 302 305-310) ou dans le brevet EP 91530 B, des allèles ou des dérivés de ces produits tels que décrits par exemple par Ju, C. et al. (J. Biol. Chem. (1987) 262 5723-5731). Les leucémies que concerne l'invention comprennent les leucémies myéloïde ou lymphoïde chroniques et les leucémies aiguës lymphoïde ou non lymphoblastique par exemple les leucémies myéloïdes d'une façon générale caractérisées par la prolifération des cellules blastiques.

L'invention a notamment pour objet l'utilisation caractérisée en ce que la leucémie est une leucémie myéloïde aiguë (LMA), diagnostiquée par les examens histologique, cytologique et biologique usuels. L'utilisation selon l'invention est destinée au traitement de la rechute de la LMA ou à son traitement préventif. L'invention décrit l'utilisation de l'$IL_2$ dans un traitement dont l'efficacité est montrée par un taux de réponse d'environ 20 % chez des malades atteints de LMA soit réfractaires à la chimiothérapie conventionnelle, soit en rechute après la chimiothérapie ou après une greffe de moelle osseuse.

L'invention a particulièrement pour objet l'utilisation caractérisée en ce que l'$IL_2$ humaine est une $IL_2$ recombinante pure. Les compositions pharmaceutiques préparées selon l'invention renferment une $IL_2$ humaine recombinante, des allèles ou des dérivés de celle-ci, telle que décrite ci-dessus, pour laquelle on utilise des techniques de purification connues de l'homme de métier, qui permettent de préparer des produits purs.

L'invention a plus particulièrement pour objet l'utilisation caractérisée en ce que l'$IL_2$ est une $IL_2$ recombinante non glycosylée sous forme réduite. L'$IL_2$ non glycosylée utilisée est notamment celle ayant la séquence de l'$IL_2$ naturelle à 133 aminoacides avec éventuellement une Méthionine N-terminale supplémentaire, dont les 3 cystéines en position 58, 105 et 125 sont sous la forme réduite, manifestant une activité biologique comparable à celle de l'$IL_2$ oxydée ayant la même séquence comportant un pont disulfure en position 58-105. Un exemple de préparation de l'$IL_2$ réduite à partir d'une souche d'E. coli est donné plus loin dans la partie expérimentale. Par forme réduite, on entend que les restes cystéines que contient l'$IL_2$ renferment un groupement sulfhydryle libre dont la détermination est faite, par exemple, par spectrophotométrie avec la dithiodipyridine comme réactif des thiols. L'activité biologique est déterminée par mesure de la prolifération de lignées cellulaires leucémiques de souris dépendantes de l'$IL_2$, CTLL-2, avec un test colorimétrique au sel de tétrazolium (Mossmann, T. J. Immunol. Meth (1983) 65 55-63).

L'activité spécifique des $IL_2$ recombinantes utilisées dans l'invention est au moins égale à 0,5 x $10^6$ U BRMP/mg, de préférence 1 x $10^7$ U BRMP/mg. L'unité d'activité $IL_2$ est définie comme la quantité qui produit 50 % de la réponse maximum dans le test. On utilise comme standard un échantillon "Biological Response Modifier Programm (BRMP) reference agent human $IL_2$ (jurkat)" fourni par le National Cancer Institute (NCI).

L'invention a spécialement pour objet l'utilisation caractérisée en ce que l'$IL_2$ est administrée à la dose de 2 à 20 x $10^6$ U/M² par injection.

La composition de la présente invention peut être administrée par exemple, par voie intraveineuse en bolus lent ou en perfusion continue, par voie musculaire ou par voie sous cutanée.

L'invention a plus spécialement pour objet l'utilisation caractérisée en ce que l'$IL_2$ est administrée par voie intraveineuse en bolus lent, à la dose de 8 x $10^6$ U/M² par injection. L'invention a tout spécialement pour objet l'utilisation caractérisée en ce que l'$IL_2$ est administrée de façon répétée au moins 2 fois par jour par cycle de plusieurs jours par semaine et caractérisée en ce que l'$IL_2$ est administrée de façon répétée pendant au moins 3 semaines non consécutives. La dose administrée, la fréquence de l'injection et la durée du traitement varient en fonction de l'état du malade. La dose journalière est généralement comprise entre 2 x $10^6$ U/M²/24h et 30 x $10^6$ U/M$_2$/24 h, de préférence de l'ordre de 20 x $10^6$ U/M²/24h par cycle de plusieurs jours chez l'adulte ou l'enfant. L'$IL_2$ est comprise dans une composition pharmaceutique, de préférence lyophilisée en flacon-dose contenant de 0,1 à 2 mg de principe actif et que l'on reconstitue avec de l'eau distillée pour injection. La solution obtenue est immédiatement diluée à l'aide d'un soluté, par exemple le chlorure de sodium à 0,9 % ou le glucose à 5 % pour l'administration en bolus lent. Par bolus lent, on entend une perfusion de courte durée par exemple de 15 mn. Selon l'utilisation préférée de l'invention, l'$IL_2$ est l'$IL_2$ recombinante réduite dont la préparation est donnée plus loin ainsi qu'un exemple de préparation pharmaceutique, la dose est de 8 x $10^6$ U/M² par injection, la fréquence de l'injection est de toutes les 8 heures (3 doses/jour) ou toutes les 12 heures (2 doses/jour) selon un cycle de 5 jours consécutifs, la durée de l'administration est de 3 semaines non consécutives, représentant environ 360 x $10^6$ U/M² et 36 mg administrés au total chez le malade par voie intraveineuse en bolus lent de 15 minutes.

Les figures ci-annexées illustrent la préparation décrite plus loin :
- la figure 1a est un chromatogramme de RP-HPLC analytique de l'extrait brut en guanidine 8M de la préparation.
- la figure 1b est un chromatogramme de RP-HPLC analytique des $IL_2$ standard réduite et oxydée.
- la figure 2 est un chromatogramme de RP-HPLC analytique de la "resolubilisation" de la préparation.
- la figure 3 est un chromatogramme de RP-HPLC de la "fraction principale" de la préparation.
- la figure 4 est un chromatogramme de RP-HPLC analytique de la fraction "59" de la préparation.

Les exemples suivants illustrent l'invention :

**Préparation de l'$IL_2$ humaine recombinante (r-h$IL_2$) réduite :**

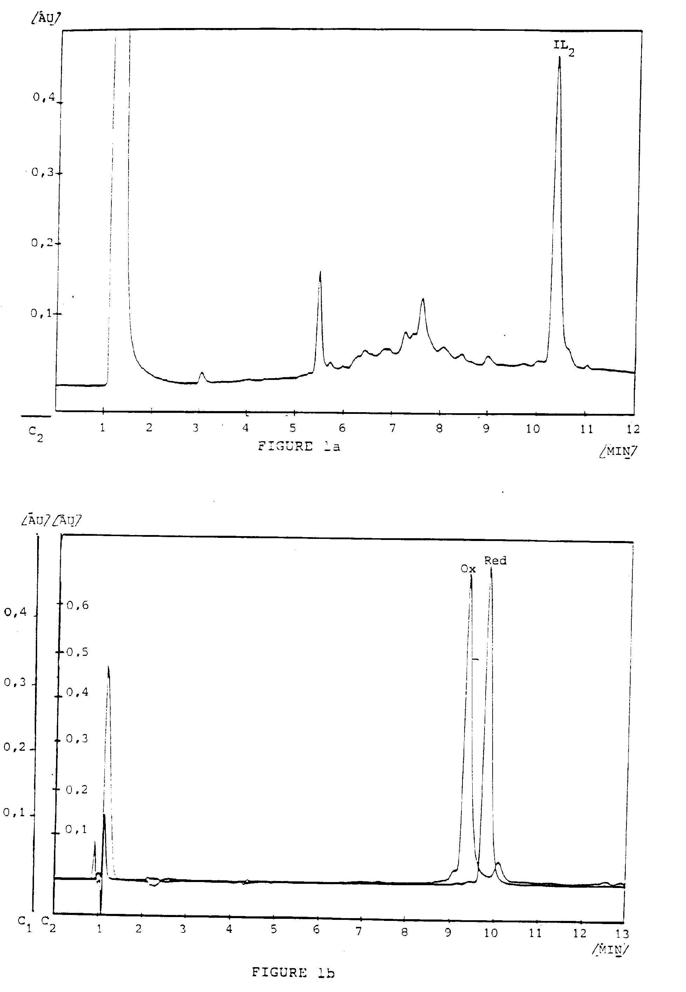

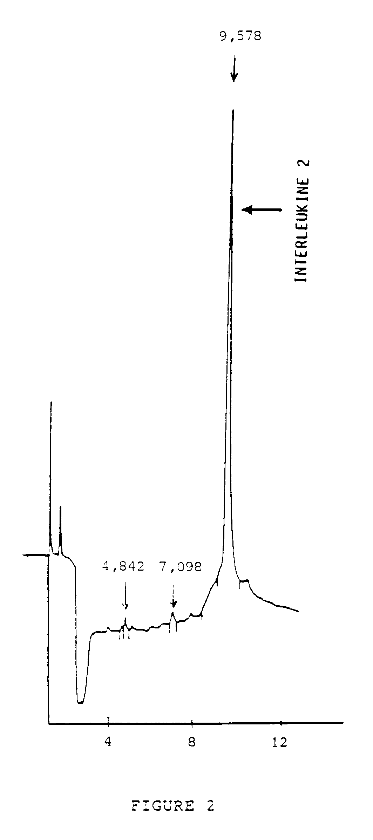

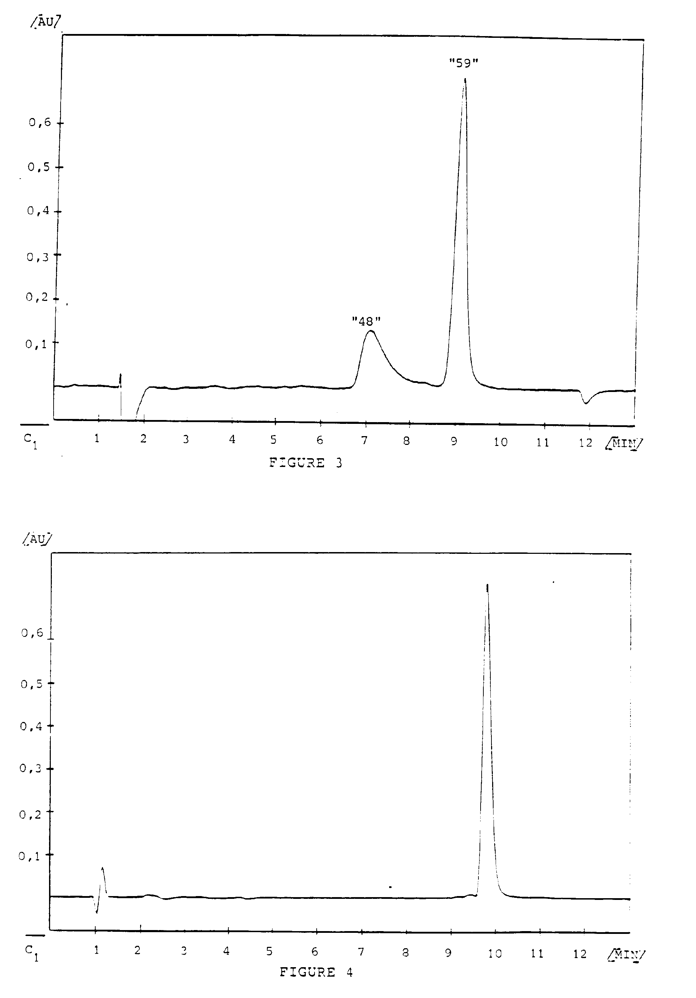

L'$IL_2$ est préparée à partir de granules qui sont obtenus par centrifugation de cultures d'une souche d'E. coli transformée à l'aide d'un plasmide comprenant la séquence codant pour l'$IL_2$ native et capable d'accumuler l'$IL_2$ sous cette forme à l'intérieur des cellules, comme décrit par exemple par Sato, T. et al. (J. Biochem. (1987) 101 525-534). Les cellules ainsi obtenues d'un fermenteur de 10 litres sont soumises à un éclatement dans un homogénéisateur Manton Gaulin. A partir des débris cellulaires isolés et lavés (90-170 g en poids humide) l'$IL_2$ est solubilisée dans 2,5 volumes d'un tampon Tris, HCl 20 mM pH 8 contenant du chlorhydrate de guanidine (Gu, HCl) 8M et du Dithiothréitol (DTT) 100 mM. La quantité d'$IL_2$ solubilisée (1,5 à 2,5 g) est estimée par RP-HPLC analytique sur une colonne C4 VYDAC (0,46 x 15 cm) 300 A°, 5 microns, au débit de 2 cm³/mn, avec un gradient linéaire d'acétonitrile (30 à 70 % sur 10 minutes) contenant 0,1 % de TFA, une détection spectrophotométrique à 280 nm ou 210 nm, dont on évalue la surface du pic à 280 nm après calibration avec un standard d'$IL_2$ (fig. 1a et fig. 1b). L'$IL_2$ est ensuite précipitée par abaissement à 2M de la concentration en Gu, HCl, en présence de DTT. Après lavage du précipité à l'aide d'une solution aqueuse de TFA à 0,1 % jusqu'à obtention d'un pH du surnageant inférieur à 5,0, l'$IL_2$ est solubilisée dans une solution aqueuse à 20 % d'acétonitrile et 0,1 % de TFA. La "resolubilisation" obtenue, qui renferme selon la RP-HPLC analytique (fig. 2) une teneur en $IL_2$ réduite supérieure à 85 %, a une activité biologique inférieure à 0,01 x $10^7$ U/mg d'$IL_2$ réduite et une teneur en groupements sulfhydryles de 2,85 SH/mole d'$IL_2$ réduite. Une fraction de la solution obtenue, correspondant à environ 200 mg d'$IL_2$ estimée par RP-HPLC analytique, est diluée de façon à ajuster la concentration en acétonitrile inférieure à 10 % dans du TFA à 0,1 %, puis appliquée à une colonne C4 VYDAC (5,7 x 30 cm). L'$IL_2$ est éluée au débit de 100 cm³/mn, à l'aide d'un gradient linéaire d'acétonitrile (30 à 80 % sur 40 minutes) contenant 0,1 % de TFA, à une concentration d'environ 60 % en acétonitrile dans un pic majeur détecté par spectrophotométrie à 280 nm et analysé par RP-HPLC. La "fraction principale" recueillie qui renferme l'$IL_2$ réduite est diluée immédiatement par 2 volumes de solution aqueuse d'acide citrique à 0,5 % et appliquée à une colonne C4 VYDAC (5,7 x 30 cm) que l'on développe avec un gradient linéaire d'isopropanol (20 à 70 % sur 40 minutes) contenant 0,5 % d'acide citrique, au débit de 50 cm$^3$/mn. L'effluent, suivi par spectrophotométrie à 280 nm, montre l'élution successive d'un pic mineur à une concentration d'environ 48 % en isopropanol (fraction "48") et d'un pic majeur à une concentration d'environ 59 % en isopropanol (fraction "59") (fig. 3). La fraction "59" est recueillie. Elle est stable à la conservation à 0°C pendant au moins 24 h, à l'abri de l'air.

Après élimination de l'isopropanol par distillation azéotropique sous vide, la fraction "59" est analysée en RP-HPLC analytique et donne un pic homogène (fig. 4) élué à environ 60 % d'acétonitrile alors que l'IL$_2$ oxydée de référence est éluée à environ 57 % d'acétonitrile (fig. 1b). La fraction "59" qui, après élimination de l'isopropanol, a une concentration en IL$_2$ supérieure à 1 mg/cm$^3$ et un pH de 3 ± 0,5, peut être conservée à +4°C, à l'abri de l'air, pendant au moins une semaine ou être immédiatement lyophilisée ou formulée pour l'obtention d'une composition pharmaceutique. La fraction "59" lyophilisée est dosée en activité biologique, selon le test in vitro de prolifération de cellules CTLL-2, et a une activité spécifique de 1,3 ± 0,5 x 10$^7$ U/mg comparable à celle de l'IL$_2$ native.

La teneur en groupements sulfhydryles libres de la fraction "59" lyophilisée, déterminée par la méthode colorimétrique à la dithiodipyridine, est de 2,94 SH/mole comparativement à 0,76 SH/mole pour l'IL$_2$ oxydée de référence.

150 à 300 mg titrés par RP-HPLC analytique de r-hIL$_2$ réduite, contenant 3 groupements SH, biologiquement active, homogène en RP-HPLC, sont obtenus dans la fraction "59" à partir d'un fermenteur de 10 litres.

## EXEMPLE 1 : Composition pharmaceutique pour perfusion.

On a réalisé une préparation pour injection par voie intraveineuse en perfusion de formule :

| | |
|---|---|
| IL$_2$ réduite | 0,5 mg |
| acide citrique | 5 mg |
| mannitol | 50 mg |
| eau stérile | 1 cm$^3$ |
| glucose à 5 % | 50 cm$^3$ |

## EXEMPLE 2 : Etude clinique dans le traitement de la LMA.

L'étude inclut des malades ayant une LMA primitive de diagnostic histologique (type M1 à M5 selon la classification FAB) pour laquelle ils ont eu une chimiothérapie d'induction suivie soit d'une chimiothérapie de maintenance, soit d'une greffe de moelle osseuse autologue (noté GDMA) et dont un diagnostic ultérieur a établi la rechute ou le caractère réfractaire à la chimiothérapie, évalué sur la présence de plus de 10 % de blastes dans deux prélèvements de moelle osseuse réalisés au moins à 1 semaine d'intervalle, par exemple à 2 semaines d'intervalle.

Les compositions d'IL$_2$ préparées selon l'invention permettent d'injecter des doses de 8 x 10$^6$ U/M$^2$, soit 0,8 mg/M$^2$ par injection, à raison d'une injection toutes les 8 heures pendant 5 jours consécutifs de la première semaine, puis la même dose que ci-dessus à raison d'une injection toutes les 12 heures pendant 5 jours consécutifs de la troisième semaine et de la cinquième semaine, par voie intraveineuse en bolus lent de 15 minutes. On utilise les compositions décrites à l'exemple 1.

L'évaluation des malades est faite avant et après le traitement à l'IL$_2$ sur les prélèvements de moelle osseuse par détermination du pourcentage de blastes et évaluation de l'état de différentiation des précurseurs médullaires.

Sur 11 malades réévalués, les réponses suivantes ont été obtenues :

| Patient | Age | Sexe | Type | Etat hématologique à l'inclusion | % de blastes médullaires après IL2 (J0 | – J8 | – J14 | – J36) | Réponse |
|---------|-----|------|------|----------------------------------|------|------|-------|------|---------|
| 1 | 68 | H | M5 | 1ère rechute sous chimio | 30 | 4 | 2 | 0 | RC |
| 2 | 17 | F | M5 | Résistance primaire | 85 | 83 | 80 | 80 | échec |
| 3 | 60 | H | M5 | 2ème rechute résistant à la chimio | 42 | 24 | 43 | 16 | échec |
| 4 | 5 | H | M5 | 3ème rechute après GDMA | 81 | 3 | ND | 0 | RC |
| 5 | 56 | H | M4 | 1ère rechute sous chimio | 13 | ND | 19 | 60 | échec |
| 6 | 25 | H | M2 | 1ère rechute sous chimio | 14 | ND | ND | 0 | échec |
| 7 | 45 | H | M4 | Résistance primaire | 75 | ND | 22 | 83 | échec |
| 8 | 28 | H | M4 | 1ère rechute après GDMA | 61 | 89 | 57 | 70 | échec |
| 9 | 53 | F | M1 | 1ère rechute résistant à la chimio | 83 | 74 | 93 | 92 | échec |
| 10 | 59 | F | M4 | 1ère rechute après GDMA | 80 | 74 | 89 | ND | échec |
| 11 | 39 | H | M1 | 1ère rechute sous chimio | 44 | 38 | 64 | 70 | échec |

EP 0 426 521 B1

Les résultats montrent 2 réponses complètes (RC), soit un taux de réponse de 20 %. Parmi les échecs, 2 malades (patients n° 3 et n° 7) présentent une diminution significative du pourcentage des blastes médullaires, suggérant une certaine efficacité de l'IL$_2$.

## Revendications

1. Utilisation d'un polypeptide ayant l'activité de l'interleukine 2 humaine pour préparer une composition pharmaceutique destinée au traitement de la rechute des leucémies ou au traitement préventif de celle-ci.

2. Utilisation selon la revendication 1 caractérisée en ce que la leucémie est une leucémie myéloïde aiguë réfractaire à la chimiothérapie, en rechute après la chimiothérapie ou en rechute après une greffe de moelle osseuse.

3. Utilisation selon la revendication 2 caractérisée en ce que le polypeptide est une IL$_2$ recombinante pure.

4. Utilisation selon la revendication 3 caractérisée en ce que l'IL$_2$ est une IL$_2$ recombinante non glycosylée sous forme réduite.

5. Utilisation selon la revendication 3 ou 4 caractérisée en ce que la composition pharmaceutique d'IL$_2$ est adaptée à être administrée à la dose de 2 à 20 x 10$^6$ U/M$^2$ par injection.

6. Utilisation selon la revendication 5 caractérisée en ce que la composition pharmaceutique d'Il$_2$ est adaptée à être administrée par voie intraveineuse en bolus lent à la dose de 8 x 10$^6$ U/M$^2$ par injection.

## Patentansprüche

1. Verwendung eines Polypeptides mit der Aktivität von humanem Interleukin 2 zur Herstellung einer pharmazeutischen Zusammensetzung, die für die Behandlung von Leukämie-Rezidiven oder für deren vorbeugende Behandlung vorgesehen ist.

2. Verwendung nach Anspruch 1, dadurch gekennzeichnet, daß die Leukamie eine akute myeloische Leukämie ist, die der Chemotheraple widersteht, und zwar im Rezidiv nach der Chemotherapie oder im Rezidiv nach einer Knochenmarktransplantation.

3. Verwendung nach Anspruch 2, dadurch gekennzeichnet, daß das Polypeptid eine reine IL$_2$-Rekombinante ist.

4. Verwendung nach Anspruch 3, dadurch gekennzeichnet, daß das IL$_2$ eine nicht glycosylierte IL$_2$-Rekombinante in reduzierter Form ist.

5. Verwendung nach Anspruch 3 oder 4, dadurch gekennzeichnet, daß die pharmazeutische Zusammensetzung von IL$_2$ an eine Verabreichung durch Injektion in einer Dosierung von 2 bis 20·10$^6$ U/M$^2$ angepaßt ist.

6. Verwendung nach Anspruch 5, dadurch gekennzeichnet, daß die pharmazeutische Zusammensetzung von IL$_2$ an eine Verabreichung auf intravenösem Weg in langsamem Bolus durch Injektion in Dosierungen von 8·10$^6$ U/M$^2$ angepaßt ist.

## Claims

1. Use of a polypeptide having the activity of human interleukin 2 for preparing a pharmaceutical composition intended for the treatment of the recurrence of leukemias or as a preventive treatment of the latter.

2. Use according to claim 1 characterized in that the leukemia is an acute myeloid leukemia refractory to chemotherapy, in recurrence after chemotherapy or in recurrence after a bone marrow transplant.

3. Use according to claim 2 characterized in that the polypeptide is a pure recombinant IL2.

4. Use according to claim 3 characterized in that the IL2 is a non-glycosylated recombinant IL2 in reduced form.

5. Use according to claim 3 or 4 characterized in that the pharmaceutical composition of IL2 is suitable to be administered at a dose of 2 to 20 x $10^6$ U/M$^2$ per injection.

6. Use according to claim 5 characterized in that the pharmaceutical composition of Il2 is suitable to be administered by intravenous route as a slow-release bolus at a dose of 8 x $10^6$ U/M$^2$ per injection.

FIGURE 1a

FIGURE 1b

FIGURE 2

FIGURE 3

FIGURE 4